# EUROPEAN PATENT SPECIFICATION

(11) **EP 1 393 727 B1**
(45) Date of publication and mention of the grant of the patent: **18.04.2007**
(21) Application number: 02728064.3
(22) Date of filing: 16.05.2002
(51) Int. Cl.: A61K 31/222, A61P 25/00, A61P 43/00

(54) **REMEDIAL AGENT FOR MYELOPATHIC DISEASE**
MITTEL ZUR BEHANDLUNG VON MYELOPATHIE
AGENT CURATIF POUR MYELOPATHIES

(30) Priority: 17.05.2001 JP 2001147230
(43) Date of publication of application: 03.03.2004
(73) Proprietor: ONO PHARMACEUTICAL CO., LTD., Osaka-shi, Osaka 541-8526 (JP)
(72) Inventor: TONAI, Takeharu, Zentsuji-shi, Kagawa 765-0001 (JP)
(74) Representative: Henkel, Feiler & Hänzel
(86) International application number: PCT/JP2002/004731
(87) International publication number: WO 2002/092074

(56) References cited:
- EP-A1- 0 347 168
- TAOKA Y. ET AL.: 'Role of neutrophil elastase in compression-induced spinal cord injury in rats' BRAIN RESEARCH vol. 799, no. 2, 1998, pages 264 - 269, XP002953586
- TONAI T. ET AL.: 'A neutrophil elastase inhibitor (ONO-5046) reduces neurologic damage after spinal cord injury in rats' J. NEUROCHEM. vol. 78, no. 5, 2001, pages 1064 - 1072, XP002953587

## Description

This invention is related to a use of N-[o-(p-pivaloyloxybenzenesulfonylamino)benzoyl]glycine monosodium salt tetrahydrate for the preparation of a pharmaceutical composition for the treatment and/or prevention of diseases due to myelopathy.

### Background

The spinal cord is damaged according to the factor of traumatic injury, for example, like cutting or compression that were caused by traffic accident, sport accident, etc., or the other factor, for example, like tumor, subdural abscess, extradural abscess, compression to hematoma or spinal canal by protrusion of bone or cartilage, destruction by infection, lesion by stopping of blood supply, injury by reperfusion after ischemia, or disease in which nerve function is changed, etc. These cause pathologic changes including hemorrhage, ischemia, edema, leukocyte infiltration, and neuronal death, damage of neurons, and symptoms of spinal pain, change of reflexes, weakness, motor palsy of limbs, respiratory muscle paralysis, loss of sensation, urinary disturbance, etc. For example, when the obstacle occurs at upper cervical spinal cord, acroparalysis and respiratory muscle paralysis are caused. When the obstacle occurs under upper cervical spinal cord, flaccid paralysis of limbs below the affected area, disappearance of perceptual and/or reflective function, or functional abnormality of urination and bowel movement may be appeared.

Moreover, paraplegia may occur in connection with spinal cord ischemia-reperfusion injury according to operation for thoracic or abdominal aortic aneurysm, dissecting aneurysm of the aorta, etc.

In general, a steroid hormone is used for the treatment for injured spinal cord caused by the factor of traumatic injury in diseases due to myelopathy. However, since it is given in large quantities, it has the problem of side effects. Moreover, in the case of ischemia of spinal cord accompanying an operation, there is still no medicine with which the spinal cord protection effect was established.

Interleukin-8 (IL-8) belongs to a family of inflammatory chemokines that share a C-X-C motif (X represents some amino acid). Especially, IL-8 is a major factor in acute inflammation. It is cytokine, which is responsible for activation of neutrophils and for neutrophil chemotaxis to sites of injury. Moreover, it became clear that four factors of CINC-1 (cytokine-induced neutrophil chmoattractant-1), CINC-2α, CINC-2β, CINC-3, which are purified from rat, have strong chemoattractant activity for neutrophils [Biochem. J., 301, 545-550 (1994)]. Based on biologic activity and peptide sequences, it is thought that these two factors, i.e., human IL-8 and rat CINCs, are relative relations.

Recently, much attention has been focused on these factors in CNS injury. For example, there is a report as shown below. Reperfusion injury after cerebral ischemia in rabbits caused neutrophil migration into the brain as well as cerebral edema in association with local production of IL-8 [Lab. Invest., 77, 119-125 (1997)]. A marked increase in CINC concentration has been detected in reperfused ischemic rat brain [Stroke, 26, 318-323 (1995)]. Further, these groups of investigators demonstrated that antibody against IL-8 or rat CINC-1 reduced cerebral edema and infarct size in cerebral reperfusion injury [Lab. Invest., 77, 119-125 (1997), Brain Res. 759, 103-111 (1997)].

These results indicate that members of the IL-8 family, representing potent neutrophil chemotactic and activating factors, are pivotal in neutrophil-mediated acute inflammation after CNS injury.

On the one hand, in the group of lysosomal hydrolases which is released from neutrophil, elastase, which belong to neutral serine proteinase locally existed in azurophil granule mainly play a part in decomposition of a connective tissue. Especially, elastase degrades elastic connective tissue by cleaving the cross-linking of elastin which directly maintains the elasticity of lung tissue etc., and by cleaving hydrophobic part of protein and degrades the cross-linking area of collagen selectively as well as elastin, and it acts on tissue proteins such as proteoglycans etc. Therefore, elastase plays an important role in metabolism of connective tissue. Elastase is inactivated by α1-proteinase inhibitor (α1-PI) and the unbalance of enzyme and inhibitor system causes the destruction of the tissue.

Moreover, it is reported that inhibitors of neutrophil elastase, Eglin C and L 658,758, are effective in spinal cord injury model in rats [Brain Research, 799, 264-269 (1998)].

### Disclosure of the invention

The provision of powerful remedy and/or preventive for diseases due to myelopathy without side effects is anticipated, and it is expected that an elastase inhibitor becomes the medical treatment and/or a prevention agent. However, of course, all the elastase inhibitor already known are not effective in myelopathic disease. In the present condition, it has not yet realized.

Under the above situation, the present inventors have energetically studied. As a result, the present inventors have found that N-[o-(p-pivaloyloxybenzenesulfonylamino)benzoyl]glycine monosodium salt tetrahydrate is effective in diseases due to myelopathy for the first time, and completed the present invention.

Accordingly, the present invention relates to a use of N-[o-(p-pivaloyloxybenzenesulfonylamino)-benzoyl]glycine monosodium salt tetrahydrate for the preparation of a pharmaceutical composition for the treatment and/or prevention of diseases due to myelopathy.

### Detailed description of the Invention

Diseases due to myelopathy are the diseases caused by spinal cord injury as a result of traumatic injury, tumor, abscess, hematoma, compression by protrusion of bone or cartilage etc., destruction by infection, ischemia, ischemia-reperfusion, or spinal cord ischemia-reperfusion injury according to operation for thoracic or abdominal aortic aneurysm, etc. Concretely, it means spinal cord injury, spinal cord ischemia-reperfusion injury or central nervous system disorders accompanying them, for example, pain of spine, disappearance of reflective function, weakness, motor palsy of limbs, paraplegia, respiratory muscle paralysis, fall of sensory or perceptual function, etc.

The compound represented by formula (I) is published in JP03-20253 (EP347168) as the compound having elastase inhibitory activities, and a process for the preparation of the compound is also described. 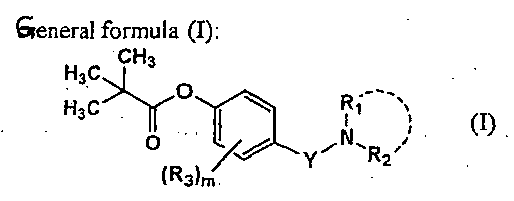 wherein Y represents sulfonyl group or carbonyl group;
R¹ and R² represent:
(i) each independently,
   (1) hydrogen atom,
   (2) C1-16 alkyl or
   (3) -X-A-(R⁴)ₙ, or
(ii) R¹, R² and nitrogen atom bonded to R¹ and R² together represent 5-12 membered mono- or bi-heterocyclic ring containing at least one nitrogen atom(s) which is substituted by -COOH;
X represents single-bond, sulfonyl group, C1-4 alkylene, C1-4 alkylene substituted by -COOH group or benzyloxycarbonyl group;
A represents C5-12 mono- or bi-carbocyclic ring or 5-12 mono- or bi-heterocyclic ring;
n represents 0 or an integer of 1 to 5;
R⁴ represents, each independently:
(1) C1-8 alkyl,
(2) C1-14 alkoxy,
(3) C1-6 alkylthio,
(4) hydroxy group,
(5) halogen atom,
(6) nitro group,
(7) trihalomethyl group,
(8) -NR⁴¹R⁴² wherein R⁴¹ and R⁴² represent, each independently, hydrogen atom or C1-4 alkyl group,
(9) tetrazole group,
(10)-SO₃H group,
(11) hydroxymethyl group,
(12) -SO₂NR⁴¹R⁴²,
(13) -Z⁴¹-COOR⁴³ wherein Z⁴¹ represents single-bond, C1-4 alkylene or C2-4 alkenylene, and R⁴³ represents hydrogen atom, C1-4 alkyl or benzyl group,
(14) -CONR⁴¹R⁴²,
(15) -COO-Z⁴²-COOR⁴³ wherein Z⁴² represents C1-4 alkylene, and R⁴³ represents hydrogen atom, C1-4 alkyl or benzyl group,
(16)-COO-Z⁴²-CONR⁴¹R⁴²,
(17) -OCO-R⁴⁵ wherein R⁴⁵ C1-8 alkyl or p-guanidinophenyl group,
(18) -CO-R⁴⁶ wherein R⁴⁶ represents C1-4 alkyl,
(19) -O-Z⁴³-COOR⁴⁵⁰ wherein Z⁴³ represents C1-6 alkylene, and R⁴⁵⁰ represents a hydrogen atom, C1-8 alkyl or p-guanidinophenyl group,
(20) 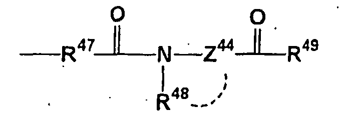 wherein -N-Z⁴⁴-CO- represents an amino acid residue, R⁴⁷ represents single-bond or C1-4 alkyl group, R⁴⁸ represents hydrogen atom or C1-4 alkyl, and R⁴⁹ represents hydroxy group, C1-4 alkoxy group, amino group, amino group substituted by one or two C1-4 alkyl group, carbamoylmethoxy or carbamoylmethoxy group substituted by one or two C1-4 alkyl group at nitrogen atom of carbamoyl group, or wherein 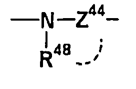 represents saturated heterocyclic ring containing one nitrogen atom and 3 to 6 carbon atoms;
R³ represents:
(1) hydroxy group,
(2) C1-6 alkyl,
(3) halogen atom,
(4) C1-4 alkoxy, or
(5) C2-5 acyloxy;
m represents 0 or an integer of 1 to 4,
a non-toxic salt of the compound, or a hydrate of the compound.

In the specification, since the compound represented by the formula (I) have elastase inhibitory, it is indicated to be effective in pulmonary emphysema, atherosclerotic cardiovascular disease and rheumatic arthritis. But a description that the compound represented by formula (I) is effective in the diseases due to myelopathy does not exist.

It is known that some relation exists between the elastase inhibitory activity and spinal cord injury. But there is no report that the compound having elastase inhibitory activity represented by formula (I) was evaluated on spinal cord injury model. Of course, there is also no report which showed efficacy. The present inventors showed that the compound represented by the formula (I) is effective against diseases due to myelopathy in the disease model due to spinal cord injury, concretely, spinal cord injury model for the first time. Moreover, the present inventor showed that the compound represented by formula (I) inhibited accumulation and infiltration of neutrophil, and it was effective in CNS injury accompanying with spinal cord injury.

Unless otherwise specified, all isomers are included in the present invention. For example, alkyl, alkoxy, alkenyl and alkynyl include straight and branched isomers. Isomers in the double bonds, rings, fused rings (E, Z, cis, trans isomers), isomers generated by the existence of asymmetric carbon atom(s) (R, S isomers, α, β isomers, enantiomers, diastereomers), optically active isomers having optically rotatory power (D, L, d, l, +, - isomers), polar isomers separated by chromatography (more polar, less polar isomers), equilibrium compounds, arbitrary ratios of these compounds, racemic mixtures are all included in the present invention.

Concretely, N-[o-(p-pivaloyloxybenzenesulfonylamino)benzoyl]glycine monosodium salt tetrahydrate is mentioned.

### Brief description of the drawings

Figure 1 represents a suppression of CINC-1 expression of the compound in the present invention on rat spinal cord injury model.
Figure 2 represents a measurement result of myeloperoxidase activity of the compound in the present invention.
Figure 3 represents an effect of suppression of the compound in the present invention against disruption of the blood-CNS barrier.
Figure 4 represents an improvement of a dyskinesia of the compound in the present invention using open field walking scale.
Figure 5 represents an improvement of a dyskinesia of the compound in the present invention using inclined-plane test.

### Best Mode for Carrying Out the Invention

The effectiveness against diseases due to myelopathy of the compound in the present invention was proved by the following experiments.

In each experiment, a compound represented by formula (X) 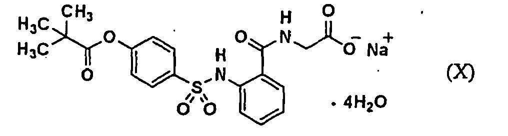 that is N-[o-(p-pivaloyloxybenzenesulfonylamino)benzoyl]glycine monosodium salt tetrahydrate, was used as test drug.

### (1) Suppression of CINC-1 expression on rat spinal cord injury model

Under anesthesia using pentobarbital (40 mg/kg of body weight), spinal cord injury model was made using Wistar rats weighing 250-300 g according to the operative procedure of Tonai et al. [J. Neurochem.. 72, 302-309 (1999)]. At various time intervals after injury, the rats were frozen, and then T13 spinal cord segment was removed for analysis of CINC-1 protein.

The test compound was dissolved in physiologic saline containing 12 mM Na₂CO₃ and injected via the femoral vein 15 min before spinal cord injury. Saline vehicle alone was administered by the same route in control animals.

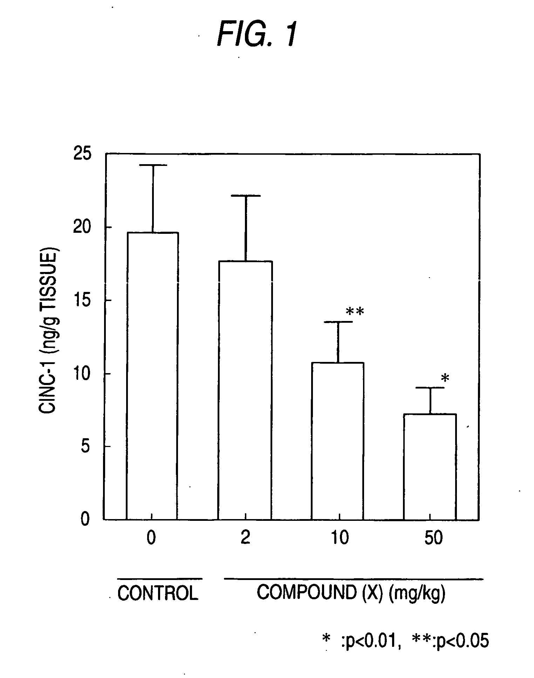

The result is shown in figure 1.

The figure 1 shows that the compound (X) suppressed in dose-dependently CINC-1 protein expression after spinal cord injury.

### (2) Assay of myeloperoxidase (MPO) activity

For assessment of accumulation and infiltration of leukocytes, we measured myeloperoxidase activity according to a modification of the method of Xu et al. (J. neurochem. 55, 907-912 (1990)). The test compound was administrated 15 minutes before spinal cord injury, and then the T13 segments 2, 4, 8, 12 and 24 h after injury were sonicated in 1 mL of 50 mM potassium phosphate buffer (pH 6.0) containing 0.5% acetyltrimethylammonium bromide. The homogenates were centrifuged at 10,000 x g for 20 min, and 0.1 mL of the supernatant was added to 0.6 mL of 50 mM potassium phosphate buffer (pH 6.0) containing 0.53 mM o-dianisidine dihydrochrolide and 0.15 mM H₂O₂. After 5 min, the change in absorbance at 460 nm was monitored with a spectrophotometer (model DU 640, Beckmann, Germany).

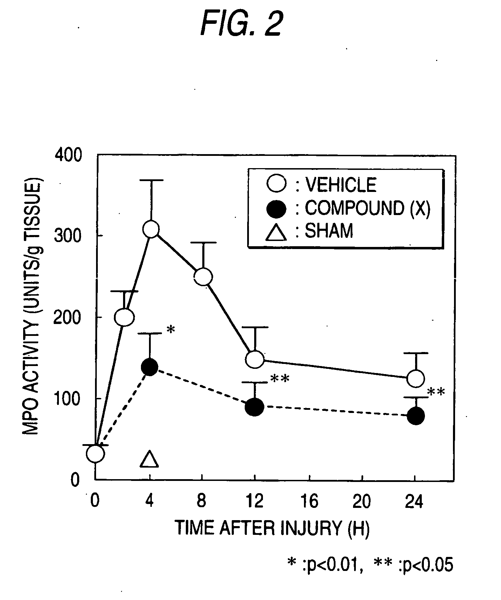

The result is shown in figure 2.

The figure 2 shows that the compound (X) suppressed myeloperoxidase activity to 46% of control 4 hours after injury, 61% of control 12 hours after injury, 64% of control 24 hours after injury, i.e., the compound (X) suppressed neutrophilic chemotaxis and activation after spinal cord injury.

### (3) Blood-CNS barrier disruption

Disruption of the blood-CNS barrier was assessed according to the method of Uyama et al. [J. Cereb. Blood Flow Metab. 8, 282-284 (1988)] with certain modifications. SCI was induced 15 min after injection of test compound (50 mg/kg). At 6 h after SCI, 4 mL/kg of 2.5% Evans blue dye solution was administered via the femoral vein. One hour later rats were anesthetized by pentobarbital (40 mg/kg) and killed. The T13 spinal cord segment was removed and homogenized in 1 mL of 50% trichloroacetic acid (w/v). After centrifugation the extracted dye was diluted fourfold with ethanol, and its fluorescence was quantified using a spectrophotometer at an excitation wavelength of 620 nm and an emission wavelength of 680 nm.

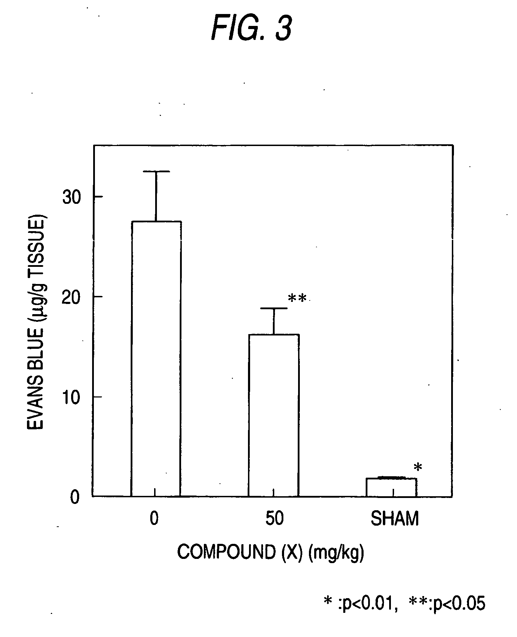

The result was shown in figure 3.

The figure 3 shows that the compound (X) suppressed Blood-CNS barrier disruption significantly.

### (4) Improvement of dyskinesia

SCI was induced 15 min after injection of test compound (50 mg/kg). Dyskinesia was assessed according to an open field walking scale (Behrmann et al., Exp. Neurol. 126, 61-75 (1994)) and an inclined-plane test (Rivlin et al., J. Neurosurg. 47, 577-581 (1977)) using rats subjected to compression injury with a 20-g weight. In the open field walking scale, animals were scored from 0 (no spontaneous movement) to 5 (normal gait) based on 5 min of observation. With the scale, the animal's capability for gross hindlimb movement was evaluated before SCI, every 2 days afterward for 2 weeks beginning on the first day, and later at 21 and 28 days after injury. In the inclined-plane test, a cork mat was fixed to the surface of a movable inclined plane. We recorded the maximal angle of inclined-plane at which the rat could maintain position for 5 minutes. This was evaluated before and at 1, 7, 14, 21 and 28 days after injury.

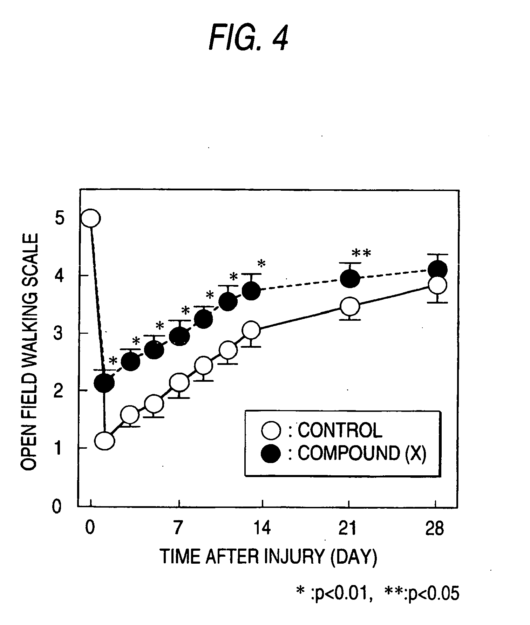

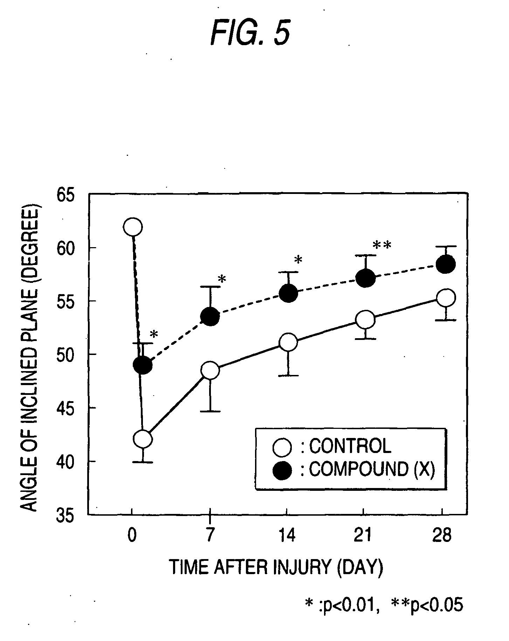

The results were shown in figure 4 and 5.

The figure 4 and 5 show that the compound (X) suppressed dyskinesia after spinal cord injury significantly.

As the above-mentioned result, since the compound (X) suppressed expression of cytokine, accumulation and infiltration of neutrophil, and disruption of the blood-CNS barrier in spine cord injury model, it is thought that the compound (X) can control an obstacle of vascular endothelial cell after spinal cord injury or various nervous disorder. Furthermore, it was also confirmed that the compound has an excellent improvement effect against dyskinesia after spinal cord injury. It can be judged that the compound represented by formula (I) in the present invention is effective in the medical treatment and/or prevention of diseases due to myelopathy.

### Toxicity

The toxicity of the compounds of the present invention is very low and therefore the compounds may be considered safe for pharmaceutical use. For example, minimum lethal dose of acute toxicity examination (intravenous administration) in rats was 450 mg/kg.

### Industrial Applicability

In animal included human, especially human, the compound represented by formula (X) of the present invention is used for prevention and/or treatment for diseases due to myelopathy. Concretely, it means spinal cord injury, spinal cord ischemia-reperfusion injury or central nervous system disorders accompanying them, for example, pain of spine, disappearance of reflective function, weakness, motor palsy of limbs, paraplegia, respiratory muscle paralysis, fall of sensory or perceptual function, etc.

In the present invention, the compound represented by formula (X) may be administered in combination with other drugs for the purpose of 1) complement and/or enhancement of preventing and/or treating effect, 2) improvement of dynamics and absorption of the compound, and lowering of dose, and/or 3) alleviation of side effect of the compound.

The compound represented by formula (X) may be administered in combination with other drugs as a composition in one drug product comprising these components, or may be administered separately. When they are administered independently, they may be administered simultaneously or with time lag. Administering with time lag includes the method of administering the compound represented by formula (X) before other drugs and vice versa; they may be administered in the same route or not.

The above combination takes effects on whichever disease treating and/or preventing effect of the compound represented by formula (X) is complemented and/or enhanced.

As other drugs to complement and/or to enhance the preventing and/or treating effect of the compound represented by the formula (X) for diseases due to myelopathy, for example, steroid drug, muscle-relaxing drug, anticholinergic agent, tricyclic antidepressant, anticonvulsant, sodium channel blocker (membrane stabilizing agent), minor tranquilizer, opioid, NMDA receptor antagonist or GABA agonist are given.

As steroid drug, for example, as external medicine, clobetasol-17-propionate, diflorasone diacetate, fluocinonide, monometasone furcate, betamethasone dipropionate, betamethasone butyrate propionate, betamethasone valerate, difluprednate, budesonide, diflucortrone valerate, amcinonide, halcinonido, dexamethasone, dexamethasone propinate, dexamethasone valerate, dexamethasone acetate, hydrocortisone acetate, hydrocortisone butyrate, hydrocortisone butyrate propionate, deprodone propionate, predonisolone valerate acetate, fluocinolone acetate, beclometasone dipropionate, triamcinolone acetonide, flumetasone pivalate, alclometasone dipropionate, clobetasone butyrate, prednisolone, beclometasone dipropionate and fludroxycortide are given.

As internal medicine or injection, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butyrate acetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate and betamethasone are given.

As inhalations, beclometasone dipropionate, fluticasone propionate, budesonide, flunisolide, triamcinolone, ST-126P, ciclesonide, dexamethasone palmitate, monometasone furoate, sodium prasterone sulfate, deflazacort, methylprednisolone suleptanate and methylprednisolone sodium succinate are given.

As muscle-relaxing drug, for example, tolperisone hydrochloride, chlorzoxazone, chlormezanone, methocarbamol, phenprobamate, pridinol mesilate, chlorphenesin carbamate, baclofen, eperisone hydrochloride, afloqualone, tizanidine hydrochloride, alcuronium chloride, suxamethonium chloride, tubocurarine chloride, dantrolene sodium, pancuronium bromide and vecuronium bromide are given.

As anticholinergic agents, for example, ipratropium bromide, oxitropium bromide, flutropium bromide, cimetropium bromide, temiverine, tiotropium bromide and revatropate (UK-112,166) are given.

As tricyclic antidepressant, for example, imipramine hydrochloride, desipramine hydrochloride, clomipramine hydrochloride, trimipramine maleate, amitriptyline hydrochloride, nortriptyline hydrochloride, lofepramine hydrochloride, amoxapine and dosulepine hydrochloride are given.

As anticonvulsant, for example, carbamazepine, phenytoin, ethotoin, mephobarbital, metharbital, primidone, trimethadione, acetylpheneturide, ethosuximide, sodium valproate, acetazolamide, clonazepam, zonisamide and diazepam are given.

As sodium channel blocker, for example, procainamide hydrochloride, ajmaline, disopyramide, disopyramide phosphate, disopyramide succinate, pirmenol hydrochloride, lidocaine hydrochloride, mexiletine hydrochloride, aprindine hydrochloride, propafenone hydrochloride, flecainide acetate, pilsicainide hydrochloride and amiodarone hydrochloride are given.

As minor tranquilizer, for example, chlordiazepoxide, diazepam, cloxazolam, oxazepam, oxazolam, medazepam, bromazepam, lorazepam, prazepam, fludiazepam, clorazepate dipotassium, mexazolam, alprazolam, flutazolam, flutoprazepam, ethyl loflazepate, clotiazepam, etizolam and hydroxyzine are given.

As opioid, for example, morphine sulfate, morphine hydrochloride, pethidine hydrochloride, fentanyl, pentazocine and buprenorphine hydrochloride are given.

As NMDA receptor antagonist, for example, ketamine and dextromethorphan hydrobromide, are given.

As GABA agonist, for example, most of minor tranquilizer, baclofen and midazolam are given.

Weight ratio of the compound represented by formula (X) and other drugs is not limited.

Other drugs may be administered as a combination of any two or more drugs.

In other drugs to complement and/or to enhance the preventing and/or treating effect of the compound represented by formula (X), drugs that not only exist now but also may be found in the future on the basis of above mechanisms are included.

When the compound represented by formula (X) which are used in the present invention, or concomitant drug combined the compound represented by formula (X) with other drugs are used for the above-described purpose, it is usually administered systemically or topically via an oral or parenteral route.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the composition of, for example, solid compositions, liquid compositions or other compositions each for oral administration, or injections, liniments suppositories, eye drops, or inhalations, each for parenteral administration.

Solid compositions for oral administration include compressed tablets, pills, capsules, powders, and granules.

Capsules include hard capsules and soft capsules.

In such solid compositions, one or more of the active substance(s) may be admixed with at least one inert diluent such as lactose, mannitol, glucose, hydroxypropyl cellulose, microcrystalline cellulose, starch, polyvinylpyrrolidone or magnesium aluminometasilicate. The compositions may comprise, in accordance with the conventional process, additives other than the inert diluent, for example, lubricants such as magnesium stearate, disintegrants such as cellulose calcium glycolate, stabilizer such as lactose, and solubilizing agent such as glutamic acid or aspartic acid. Tablets or pills may be coated with a film of a gastric soluble or enteric substance such as sucrose, gelatin, hydroxypropyl cellulose or hydroxypropyl methylcellulose phthalate, or with two or more layers, if necessary. Furthermore, capsules made of a substance which can be absorbed in the body, for example, gelatin, are included.

Liquid compositions for oral administration include pharmaceutically acceptable emulsions, solutions, syrups and elixirs. Such liquid compositions comprise one or more of the active substance(s) and an ordinarily employed inert diluent(s) (for example, purified water or ethanol) dissolving the substance(s) therein. The compositions may comprise, in addition to the inert diluent, an adjuvant such as humectants or suspending agents, sweetening agents, flavoring agents, aromatic agents and antiseptics.

The other compositions for oral administration include sprays which comprise one or more active substance(s) and are formulated in a manner known per se in the art. The compositions may comprise, in addition to an inert diluent, a stabilizer such as sodium bisulfite and an isotonization buffer such as sodium chloride, sodium citrate or citric acid. The preparation process of sprays is described in detail in, for example, U.S. Patent Nos. 2,868,691 and 3,095,355.

In the present invention, injections for parenteral administration include sterile aqueous and/or non-aqueous solutions, suspensions and emulsions. The aqueous solutions or suspensions include, for example, distilled water for injection and saline. The non-aqueous solutions or suspensions include propylene glycol, polyethylene glycol, vegetable oils such as olive oil, alcohol such as ethanol and Polysorbate 80 (trade mark). Furthermore, sterile aqueous and non-aqueous solutions, suspensions, and emulsions may be used in combination. Such compositions may additionally comprise adjuvants such as antisaptic, humectant, emulsfier, dispersant, stabilizer (such as lactose) and solubilizing agent (such as glutamic acid and aspartic acid). They are sterilized by filtration through a bacteria retaining filter, the addition of a sterilizer, or irradiation. Also, a sterile solid composition is prepared and then, for example, a freeze-dried product may be dissolved in sterilized or sterile distilled water for injection or another sterile solvent before use.

The dosage form of eye drops for parenteral administration include ophthalmic solutions, ophthalmic suspensions, ophthalmic emulsions, ophthalmic solutions dissolved before use and eye ointments.

Such eye drops are prepared in a known method. For example, an ophthalmic solution is prepared by selecting proper additives from an isotonizing agent (such as sodium chloride or concentrated glycerin), a buffering agent (such as sodium phosphate or sodium acetate), a surfactant (such as "Polysorbate 80" (trade name), polyoxyl 40 stearate or polyoxyethylene hydrogenated castor oil), a stabilizer (such as sodium citrate or edetate sodium) and an antiseptic (such as benzalkonium chloride or p-aminobenzonic acid), if necessary. They are sterilized or subjected to aseptic manipulation in the final step.

The dosage of inhalations for parenreral administration include aerosol, powders for inhalation or liquids for inhalation. The liquids for inhalation may be dissolved or suspended in water or the other appropriate solvent as needed.

Such inhalations are prepared in a known method. For example, a liquid for inhalation is prepared by selecting proper additives from an antiseptic (such as benzalkonium chloride or p-aminobenzonic acid), a coloring agent, a buffering agent (such as sodium phosphate or sodium acetate), an isotonizing agent (such as sodium chloride or concentrated glycerin), thickening agent (such as carboxyvinyl polymer), or an accelerator of absorption, if necessary.

A powder for inhalation is prepared by selecting proper additives from a lubricant agent (such as stearin acid and the salt thereof), a binding agent, (such as starch, dextrin), a diluting agent (such as lactose, cellulose), a coloring agent, an antiseptic (such as benzalkonium chloride or p-aminobenzonic acid), an accelerator of absorption, if necessary.

In case of administration of liquid for inhalation, spray (atomizer, nebulizer) is usually used and in case of administration of powder for inhalation, inhalation administration apparatus for powder agents is usually used.

The other compositions for parenteral administration include liquids for external use, ointments, endermic liniments, suppositories for intrarectal administration and pessaries for vaginal administration which comprise one or more of the active substance(s) and may be prepared by methods known per se.

### Formulation example 1

The following components were admixed in a conventional manner, punched out to give 100 tablets each containing 50 mg of active ingredient.
- N-[o-(p-pivaloyloxybenzenesulfonylamino)benzoyl]glycine monosodium salt tetrahydrate 5.0g
- carcium carboxymethyl cellulose (disintegrant) 0.2g
- magnesium stearate(lubricant) 0.1g
- microcrystalline cellulose 4.7g

### Formulation example 2

The following components were admixed in a conventional technique. The solution was sterilized in a conventional technique, filled in ampoules 5 ml each and freeze-dried over in a conventional technique to give 100 ampoules each containing 20 mg of active ingredient.
- N-[o-(p-pivaloyloxybenzenesulfonylamino)benzoyl]glycine monosodium salt tetrahydrate 2.0g
- mannitol 20g
- distilled water 500 mL

## Claims

1. Use of N-[o-(p-pivaloyloxybenzenesulfonyl-amino)benzoyl]glycine monosodium salt tetrahydrate for the preparation of a pharmaceutical composition for the treatment and/or prevention of diseases due to myelopathy.

2. The use according to claim 1, wherein the disease due to myelopathy is spinal cord injury or central nervous system disorders accompanying it.

3. The use according to claim 1, wherein the disease due to myelopathy is spinal cord ischemia-reperfusion injury or central nervous system disorders accompanying it.

## Patentansprüche

1. Verwendung von N-[o-(p-Pivaloyloxybenzolsufonylamino)-benzoyl]glycin-mononatriumsalz-tetrahydrat zur Herstellung einer pharmazeutischen Zusammensetzung zur Behandlung und/ oder Prävention von Erkrankungen aufgrund von Myelopathie.

2. Verwendung nach Anspruch 1, wobei die Erkrankung aufgrund von Myelopathie eine Wirbelsäulenläsion oder diese begleitende Störungen des Zentralnervensystems sind.

3. Verwendung nach Anspruch 1, wobei die Erkrankung aufgrund von Myelopathie eine Wirbelsäulen-Ischämie/Reperfusionsläsion oder diese begleitende Störungen des Zentralnervensystems sind.

## Revendications

1. Utilisation d'un tétrahydrate de sel monosodique de N-[o-(p-pivaloyloxybenzènesulfonylamino)benzoyl]glycine pour la préparation d'une composition pharmaceutique destinée au traitement et/ou à la prévention de maladies dues à une myélopathie.

2. Utilisation selon la revendication 1, dans laquelle la maladie due à une myélopathie est une blessure de la moelle épinière ou des troubles du système nerveux central l'accompagnant.

3. Utilisation selon la revendication 1, dans laquelle la maladie due à une myélopathie est une blessure d'ischémie-reperfusion de la moelle épinière ou des troubles du système nerveux central l'accompagnant.
